# EUROPÄISCHE PATENTANMELDUNG

(11) **EP 1 985 279 A1**
(43) Veröffentlichungstag der Anmeldung: **29.10.2008**
(21) Anmeldenummer: 07008475.1
(22) Anmeldetag: 26.04.2007
(51) Int. Cl.: A61K 8/02, A61K 8/368, C07C 63/06

(54) **Kosmetische Zusammensetzungen enthaltend Ester auf Basis von 2-Propylheptanol**

(71) Anmelder: Cognis IP Management GmbH, 40589 Düsseldorf (DE)
(72) Erfinder: Dierker, Dr. Markus, 40597 Düsseldorf (DE)

(57) **Zusammenfassung**

Gegenstand der Erfindung ist die Verwendung von Estern des 2-Propylheptanols mit Benzoesäure und/oder Benzoesäurederivaten in kosmetischen und/oder pharmazeutischen Zubereitungen. Die Verbindungen zeichnen sich durch ihre besonders leichte Sensorik aus.

## Beschreibung

### Gebiet der Erfindung

Die Erfindung betrifft die Verwendung von Estern des 2-Proplyheptanols mit Benzoesäure und/oder Benzoesäurederivaten in kosmetischen und/oder pharmazeutischen Zubereitungen sowie spezifische Ester und ein Verfahren zu deren Herstellung.

### Stand der Technik

Im Bereich kosmetischer Emulsionen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper und Emulgator-Gemische entwickelt und getestet. Die Verwendung von Esterölen in der Kosmetik ist seit langem bekannt. Wegen ihrer Bedeutung werden auch kontinuierlich neue Verfahren zu deren Herstellung entwickelt. Insbesondere verzweigte Esteröle vermitteln ein "leichteres" Hautgefühlt und werden daher intensiv untersucht. Der Einsatz von 2-Methyl-1,3-propanediolmonoestem ist beispielsweise Gegenstand der DE 101 60 681, der Einsatz von 2-Methyl-1,3-propanedioldiestem wird in der DE 101 60 682 beschrieben.

Aufgabe der vorliegenden Erfindung war es, neue, vorzugsweise bei 20°C flüssige Esteröle für kosmetische Applikationen zur Verfügung zu stellen, die bezüglich der sensorischen Eigenschaften (Leichtigkeit, "nicht-fettendes Hautgefühl", Weichheit, Spreitvermögen, Absorption, Verteilbarkeit, Öligkeit) über ein verbessertes Profil verfügen und in eine Vielzahl kosmetischer Formulierungen einarbeitbar sind. Hierbei waren auch die Hydrolysestabilität der Ester sowie die Formulierbarkeit der Ester bei niedrigem pH-Wert von Interesse. Weiterhin sollten die Ester sowohl in W/O als auch in O/W Formulierungen einarbeitbar sein. Weiterhin sollten die Ester insbesondere mit kristallinen UV-Filtem, Pigmenten, Antiperspirantien Salzen sowie Silikonen kompatibel sein. Überraschenderweise wurde gefunden, dass Ester des 2-Propylheptanols zu sensorisch leichten Produkten führen. Einige dieser Ester und deren Herstellung sind aus der DE 103 05 562 bekannt, allerdings für einen völlig anderen Applikationszweck, als Polymeradditive. WO 2006/097235 beschreibt Ester von 2-Propylheptanol mit linearen oder verzweigten Carbonsäuren. Diese Ester sind allerdings bei der Formulierung von Zubereitungen, welche UV-Filtersubstanzen enthalten nachteilig. Hier ist vor allem die unbefriedigende Löslichkeit der UV-Filter ein limitierender Faktor.

Die Aufgabe der vorliegenden Erfindung bestand deshalb darin, gegenüber dem Stand der Technik verbesserte Ester bereit zu stellen, insbesondere solche, welche zusammen mit UV-Filtersubstanzen gut formulierbar sind und gleichzeitig hinsichtlich der Sensorik keine Nachteile gegenüber dem Stand der Technik aufweisen.

### Beschreibung der Erfindung

Gegenstand der Erfindung ist die Verwendung von Estern des 2-Propylheptanols mit Benzoesäure und/oder Benzoesäurederivaten in kosmetischen und/oder pharmazeutischen Zubereitungen.

Überaschenderweise sind Ester mit Propylverzweigung in der Alkylkette besonders gut für kosmetische Formulierungen geeignet, insbesondere für Formulierungen, bei denen es auf ein "leichtes" Hautgefühl ankommt. Die Ester lassen sich sehr gut in verschiedene Formulierungen einarbeiten. Je nach Kettenlänge, Verzweigung und Anzahl der Doppelbindungen erhält man flüssige Substanzgemische, die sich entsprechend als Ölkörper oder Konsistenzgeber eignen. Erfindungsgemäß kann ein einziger 2-Propylheptyl-Ester eingesetzt werden oder ein beliebiges Gemisch verschiedener Ester.

Gegenstand der Erfindung ist insbesondere die Verwendung von Estern des 2-Propylheptanols mit Benzoesäure und/oder Benzoesäurederivaten in kosmetischen und/oder pharmazeutischen Zubereitungen zur Benetzung oder Imprägnierung oder Beschichtung von Gebrauchs- und/oder Hygienetüchem, die zur Körperreinigung und/oder zur Körperpflege eingesetzt werden.

In einer bevorzugten Ausführungsform der Erfindung werden Ester verwendet deren Gesamt C Zahl kleiner gleich 24, bevorzugt kleiner gleich 22 beträgt.

Erfindungsgemäß sind Ester des 2-Propylheptanols mit Benzoesäure und/oder Benzoesäurederivaten. Als Benzoesäurederivate geeignet sind
- einfach oder mehrfach carboxysubstituierte Benzoesäuren, wie beispielsweise Benzoldicarbonsäuren, wie 1,2-Benzoidicarbonsäure, 1,3-Benzoldicarbonsäure, 1,4-Benzoldicarbonsäure (Terephtalsäure). Diese können gegebenenfalls alkyl- oder hydroxylsubstituiert sein.
- einfach oder mehrfach alkylsubstituierte Benzoesäuren wie z.B. 2-Methylbenzoesäure (= o-Toluylsäure), 3- Methylbenzoesäure(= m-Toluylsäure), 4-Methylbenzoesäure(= p-Toluylsäure), 2,4-Dimethylbenzoesäure, 2-Ethylbenzoesäure etc.
- einfach oder mehrfach hydroxysubstituierte Benzoesäure, wie z.B. 2-Hydroxybenzoesäure (Salicylsäure), 3-Hydroxybenzoesäure, 4-Hydroxybenzoesäure, 2,4,Dihydroxybenzoesäure
- hydroxy- und alklysubstituierte Benzoesäuren, wie beispielsweise 2-Methyl-3-Hydroxylbenzoesäure
- durch Acylierung von hydroxysubstiuierten Benzoesäuren erhältlichen Derivaten, wie beispielsweise Acetylsalicylsäure.

In einer bevorzugten Ausführungsform der Erfindung werden als Benzoesäurederivate eingesetzt, Verbindungen ausgewählt aus der Gruppe bestehend aus methylsubstituierter Benzoesäure, hydroxysubstituierter Benzoesäure, carboxysubstituierter Benzoesäure, durch Acylierung von hydroxysubstituierten Benzoesäuren erhältlichen Derivaten.

Der Begriff "Ester von 2-Propylheptanol mit Benzoldicarbonsäuren" umfasst sowohl Diester der Benzoldicarbonsäure mit 2-Propylheptanol, also beispielsweise Di-2-Propylheptyl-1,4-benzolsäurediester [Di-2-Propylheptyl-terephtalsäure] als auch Monoester, wie z.B. 2-Propylheptyl-1,4-benzolsäureester, in der nur eine Säuregruppe der Benzoldicarbonsäure verestert ist und in der die zweite Säuregruppe frei vorliegt. Umfasst sind auch gemischte Ester, in denen eine Säuregruppe der Benzoldicarbonsäure mit 2-Propylheptanol verestert ist und die zweite Säuregruppe mit einem weiteren Alkohol verestert ist. In einer Ausführungsform der Erfindung werden gemischte Ester von Benzoldicarbonsäuren (insbesondere von Benzol-1,4-dicarbonsäuren) und 2-Propylheptanol und einem weiteren Alkohol, ausgewählt aus der Gruppe bestehend aus 3-Methyl-2-propylhexanol, 4-Methyl-2-propylhexanol und 5-Methyl-2-propylhexanol eingesetzt.

In einer Ausführungsform der Erfindung werden die gemischten Ester erhalten durch Umsetzung der entsprechenden Benzoldicarbonsäure mit einem Gemisch aus 2-Propylheptanol, 3-Methyl-2-propylhexanol, 4-Methyl-2-propylhexanol und 5-Methyl-2-propylhexanol.

In einer weiteren Ausführungsform werden eingesetzt gemischte Ester von Benzoldicarbonsäuren und 2-Propylheptanol und einem weiteren Alkohol der allgemeinen Formel R-OH, wobei R für einen linearen oder verzweigten, gesättigten oder ungesättigten Alkylrest mit 1 bis 12 C-Atomen steht.

In einer weiteren Ausführungsform werden eingesetzt gemischte Ester von Benzoldicarbonsäuren und 2-Propylheptanol und einem weiteren Alkohol der allgemeinen Formel R-OH, wobei R für einen gesättigten, linearen oder verzweigten, Alkylrest mit 1 bis 12 C-Atomen steht.

In einer bevorzugten Ausführungsform werden verwendet gemischte Ester von Benzoldicarbonsäuren und 2-Propylheptanol und einem weiteren Alkohol, wobei der weitere Alkohol ausgewählt ist aus der Gruppe bestehend aus Methanol, Ethanol, Propanol, Isopropanol Butanol, Isobutanol, Pentanol, Hexanol, Isohexanol, Octanol, Decanol oder Dodecanol.

Ein weiterer Gegenstand der Erfindung sind Ester von 2-Propylheptanol mit Benzoesäure und/oder Benzoesäurederivaten. Eine bevorzugten Ausführungsform betrifft Ester ausgewählt aus der Gruppe bestehend aus 2-Propylheptyl-benzoesäureester, Di-[2-Propylheptyl-1,4-benzoldicarbonsäurediester, 2-Propylheptyl-1,4-benzolddicarbonsäureester, 2-Propylheptyl-2-Hydroxybenzoesäureester, 2-Propylheptyl-2-methylbenzoesäureester, 2-Propylheptyl-3-methylbenzoesäureester, 2-Propylheptyl-4-methyl-benzoesäureester, 2-Propylheptyl-2-Acetoxylbenzoesäureester (=2-Propylheptyl-acetylsalicylsäureester).

Die Erfindung umfasst sowohl einzelne Ester aus auch Mischungen von verschiedenen Estern.

Gegenstand der Erfindung sind Zusammensetzungen, enthaltend einen Ester von 2-Propylheptanol mit Benzoesäure und/oder Benzoesäurederivaten und mindestens einen weiteren Ester derselben Säure mit einem Alkohol ausgewählt aus der Gruppe bestehend aus 3-Methyl-2-Propylhexanol, 4-Methyl-2-Propylhexanol oder 5-Methyl-2-Propylhexanol.

Ein Gegenstand der Erfindung betrifft somit beispielsweise Zusammensetzungen, enthaltend 2-Propylheptyl-benzoesäureester und mindestens einen weiteren Ester ausgewählt aus der Gruppe bestehend aus 3-Methyl-2-propylhexyl-benzoeesäureester, 4-Methyl-2-propylhexyl-benzoesäureester, 5-Methyl-2-propylhexyl-benzoesäureester und Mischungen daraus.

In einer bevorzugten Ausführungsform der Erfindung enthaltend diese Zusammensetzungen
- 80 bis 99,99 Gew.-% des 2-Propylheptylesters
- 0,01 bis 20 Gew.-% des entsprechenden Methyl-2-Propylhexylesters.

Diese Zusammensetzungen eignen sich in kosmetischen und/oder pharmazeutischen Zubereitungen.

Gegenstand ist auch ein Verfahren zur Herstellung der erfindungsgemäßen Ester, wobei man eine Mischung enthaltend 2-Propylheptanol und die entsprechende Säure umsetzt.

Das erfindungsgemäße Verfahren umfasst ebenfalls die Herstellung von Estergemischen, bei dem man 2-Propylheptanol zusammen mit den entsprechenden Säuregemischen umsetzt.

Das erfindungsgemäße Verfahren umfasst ebenfalls die Herstellung von Zusammensetzungen gemäß Anspruch 5, bei dem man ein Gemisch aus 2-Propylheptanol und mindestens einem Alkohol ausgewählt aus der Gruppe bestehend aus 3-Methyl-2-propylhexanol, 4-Methyl-2-propylhexanol oder 5-Methyl-2-propylhexanol und die entsprechende Säure umsetzt.

In einer bevorzugten Ausführungsform der Erfindung setzt man die Mischung enthaltend Alkohol und die entsprechende Säure unter Zusatz eines Veresterungskatalysators um.

In einer bevorzugten Ausführungsform wird die Mischung enthaltend Alkohol und die entsprechende Säure erhitzt, das entstehende Wasser kontinuierlich abgeführt und das Rohprodukt danach destilliert. Das Verfahren kann unter Zusatz eines Veresterungskatalysators, z.B. säurekatalytisch oder basenkatalytisch durchgeführt werden. In einer bevorzugten Ausführungsform wird das Verfahren ohne Zusatz von Lösungsmitteln durchgeführt, vorzugsweise mit Edukten, die möglichst wasserfrei sind. In einer bevorzugten Ausführungsform des Verfahrens wird ein Zinn-Katalysator einsetzt. Als Zinn-Katalysatoren geeignet sind beispielsweise Zinnoxalat (z. B. Fascat^{®} 2001), Zinnoxid (SnO, Fascat^{®} 2000) sowie Zinn-IV-Katalysatoren wie Dibutylzinndiacetat Fascat^{®} 4200), Dibutlyzinnoxid (Fascat^{®} 4201), und Dibutylzinnlaurat (Fascat^{®} 4202) oder Zinnoxid (SnO), die ehemals von Atofina und gegenwärtig von Arkema vermarktet werden.

Vorzugsweise wird die Veresterung bei Temperaturen zwischen 100- 300°C, insbesondere 200 - 250°C durchführt.

In einer weiteren Ausführungsform wird als Katalysator mindestens ein Enzym eingesetzt. Als Enzym eignen sich alle dem Fachmann bekannten Enzyme oder Enzymmischungen, welche in der Lage sind die Veresterung von Alkohol und Säure zu katalysieren, exemplarisch seinen genannt Lipasen, Acyltransferasen und Esterasen. Die enzymatisch katalysierte Veresterung wird üblicherweise bei Temperaturen von 20 bis 100 °C, vorzugsweise 40 bis 80 °C durchgeführt.

Gegenstand der Erfindung ist ein Verfahren zur Herstellung der erfindungsgemäßen Ester, wobei man eine Mischung enthaltend 2-Propylheptanol und den Methylester der entsprechenden Säure unter Zusatz eines Umesterungskatalysators umsetzt.

Das erfindungsgemäße Verfahren umfasst ebenfalls die Herstellung von Estergemischen, bei dem man 2-Propylheptanol zusammen mit den entsprechenden Mischungen der Methylestern der Säuren unter Zusatz eines Umesterungskatalysators umsetzt.

Das erfindungsgemäße Verfahren umfasst ebenfalls die Herstellung von Zusammensetzungen gemäß Anspruch 5, bei dem man eine Gemisch aus 2-Propylheptanol und mindestens einem Alkohol ausgewählt aus der Gruppe bestehend aus 3-Methyl-2-propylhexanol, 4-Methyl-2-propylhexanol oder 5-Methyl-2-propylhexanol und den Methylester der entsprechenden Säure unter Zusatz eines Umesterungskatalysators umsetzt.

In einer bevorzugten Ausführungsform wird die Mischung enthaltend Alkohol und den Methylester der entsprechende Säure unter Zusatz des Veresterungskatalysators erhitzt, das entstehende Wasser kontinuierlich abgeführt und das Rohprodukt danach destilliert. In einer bevorzugten Ausführungsform wird das Verfahren ohne Zusatz von Lösungsmitteln durchgeführt, vorzugsweise mit Edukten, die möglichst wasserfrei sind.

Vorzugsweise wird die Veresterung bei Temperaturen zwischen 100- 300°C, insbesondere 200 - 250°C durchführt. Als Umesterungskatalysator können alle dem Fachmann bekannten Umesterungskatalysatoren eingesetzt, werden, vorzugsweise wird als Umesterungskatalysator Natriummethylat oder Tetraalkyltitanat eingesetzt.

In einer weiteren Ausführungsform wird als Katalysator mindestens ein Enzym eingesetzt. Als Enzym eignen sich alle dem Fachmann bekannten Enzyme oder Enzymmischungen, welche in der Lage sind die Umesterung von Alkohol und Säuremethylester zu katalysieren, exemplarisch seinen genannt Lipasen, Acyltransferasen und Esterasen. Die enzymatisch katalysierte Veresterung wird üblicherweise bei Temperaturen von 20 bis 100 °C, vorzugsweise 40 bis 80 °C durchgeführt.

### Ester von 3-Methyl-2-propylhexanol mit Benzoesäure und/oder Benzoesäurederivaten

Ein weiterer Gegenstand der Erfindung betrifft Ester von 3-Methyl-2-propylhexanol mit Benzoesäure und/oder Benzoesäurederivaten sowie ihre Verwendung in kosmetischen und/oder pharmazeutischen Zubereitungen.

Ein Gegenstand der Erfindung ist 3-Methyl-2-propylhexyl-benzoesäure. Ein Gegenstand der Erfindung ist Di-[3-Methyl-2-propylhexyl]-1,4-benzolsäurediester.

Die im folgenden verwendeten Begriffe "Ester von X-Methyl-2-propylhexanol mit Benzoldicarbonsäuren" umfassen sowohl Diester der Benzoldicarbonsäuren mit dem jeweiligen Methyl-2-Propylhexanol, also beispielsweise Di-3-Methyl-2-Propylhexyl-1,4-benzoldicarbonsäurediester oder Di-5-Methyl-2-Propylhexyl-1,4-benzoldicarbonsäurediester als auch Monoester, wie z.B. 3-Methyl-2-Propylheptyl-1,4-Benzolsäuremonoester als auch gemischte Ester, in denen eine Säuregruppe der Benzoldiicarbonsäure mit dem jeweiligen Methyl-2-Propylhexanol verestert ist und die zweite Säuregruppe der Benzoldicarbonsäure mit einem zweiten Alkohol verestert ist. Der zweite Alkohol kann ausgewählt werden aus 3-Methyl-2-propylhexanol, 4-Methyl-2-propylhexanol und 5-Methyl-2-propylhexanol, wobei dieser zweite Alkohol von dem ersten Methyl-2-Propylhexanol verschieden sein muss. In einer weiteren Ausführungsform der gemischten Ester wird der zweite Alkohol ausgewählt aus der Gruppe die gebildet wird von Methanol, Ethanol, Propanol, Isopropanol Butanol, Isobutanol, Pentanol, Hexanol, Isohexanol, Octanol, Decanol und Dodecanol.

### Ester von 4-Methyl-2-propylhexanol mit Benzoesäure und/oder Benzoesäurederivaten

Ein weiterer Gegenstand der Erfindung betrifft Ester von 4-Methyl-2-propylhexanol mit Benzoesäure und/oder Benzoesäurederivaten sowie ihre Verwendung in kosmetischen und/oder pharmazeutischen Zubereitungen.

Ein Gegenstand der Erfindung ist 4-Methyl-2-propylhexyl-benzoesäure. Ein Gegenstand der Erfindung ist Di-[4-Methyl-2-propylhexyl]-1,4-benzolsäurediester.

### Ester von 5-Methyl-2-propylhexanol mit Benzoesäure und/oder Benzoesäurederivaten

Ein weiterer Gegenstand der Erfindung betrifft Ester von 5-Methyl-2-propylhexanol mit Benzoesäure und/oder Benzoesäurederivaten sowie ihre Verwendung in kosmetischen und/oder pharmazeutischen Zubereitungen.

Ein Gegenstand der Erfindung ist 5-Methyl-2-propylhexyl-benzoesäure. Ein Gegenstand der Erfindung ist Di-[5-Methyl-2-propylhexyl]-1,4-benzolsäurediester.

Gegenstand ist auch ein Verfahren zur Herstellung von Estern von 3-Methyl-2-propylhexanol mit Benzoesäure und/oder Benzoesäurederivaten, wobei man eine Mischung enthaltend 3-Methyl-2-Propylhexanol und die entsprechende Säure (Carbonsäure und/oder Dicarbonsäure) umsetzt. Das erfindungsgemäße Verfahren umfasst ebenfalls die Herstellung von Estergemischen, bei dem man 3-Methyl-2-Propylhexanol zusammen mit den entsprechenden Säuregemischen umsetzt. Das erfindungsgemäße Verfahren umfasst ebenfalls die Herstellung von gemischten Estern, bei dem man 3-Methyl-2-Propylhexanol und einen weiteren Alkohol zusammen mit der entsprechenden Dicarbonsäure umsetzt.

Gegenstand ist auch ein Verfahren zur Herstellung von Estern von 4-Methyl-2-propylhexanol mit Benzoesäure und/oder Benzoesäurederivaten, wobei man eine Mischung enthaltend 4-Methyl-2-Propylhexanol und die entsprechende Säure (Carbonsäure und/oder Dicarbonsäure) umsetzt. Das erfindungsgemäße Verfahren umfasst ebenfalls die Herstellung von Estergemischen, bei dem man 4-Methyl-2-Propylhexanol zusammen mit den entsprechenden Säuregemischen umsetzt. Das erfindungsgemäße Verfahren umfasst ebenfalls die Herstellung von gemischten Estern, bei dem man 4-Methyl-2-Propylhexanol und einen weiteren Alkohol zusammen mit der entsprechenden Dicarbonsäure umsetzt.

Gegenstand ist auch ein Verfahren zur Herstellung von Estern von 5-Methyl-2-propylhexanol mit Benzoesäure und/oder Benzoesäurederivaten, wobei man eine Mischung enthaltend 5-Methyl-2-Propylhexanol und die entsprechende Säure (Carbonsäure und/oder Dicarbonsäure) umsetzt. Das erfindungsgemäße Verfahren umfasst ebenfalls die Herstellung von Estergemischen, bei dem man 5-Methyl-2-Propylhexanol zusammen mit den entsprechenden Säuregemischen umsetzt. Das erfindungsgemäße Verfahren umfasst ebenfalls die Herstellung von gemischten Estern, bei dem man 5-Methyl-2-Propylhexanol und einen weiteren Alkohol zusammen mit der entsprechenden Dicarbonsäure umsetzt.

In einer bevorzugten Ausführungsform der Erfindung setzt man die Mischung enthaltend Alkohol (5-Methyl-2-propylhexanol, 4-Methyl-2propylhexanol und/oder 3-Methyl-2-propylhexanol, bzw. gegebenenfalls den weiteren Alkohol) und die entsprechende Säure unter Zusatz eines Veresterungskatalysators um.

In einer bevorzugten Ausführungsform wird die Mischung enthaltend Alkohol (5-Methyl-2-propylhexanol, 4-Methyl-2propylhexanol und/oder 3-Methyl-2-propylhexanol bzw. gegebenenfalls den weiteren Alkohol) und die entsprechende Säure erhitzt, das entstehende Wasser kontinuierlich abgeführt und das Rohprodukt danach destilliert. Das Verfahren kann unter Zusatz eines Veresterungskatalysators, z.B. säurekatalytisch oder basenkatalytisch durchgeführt werden. In einer bevorzugten Ausführungsform wird das Verfahren ohne Zusatz von Lösungsmitteln durchgeführt, vorzugsweise mit Edukten, die möglichst wasserfrei sind. In einer bevorzugten Ausführungsform des Verfahrens wird ein Zinn-Katalysator einsetzt. Als Zinn-Katalysatoren geeignet sind beispielsweise Zinnoxalat (z. B. Fascat^{®} 2001), Zinnoxid (SnO, Fascat^{®} 2000) sowie Zinn-IV-Katalysatoren wie Dibutylzinndiacetat Fascat^{®} 4200), Dibutlyzinnoxid (Fascat^{®} 4201), und Dibutylzinnlaurat (Fascat^{®} 4202) oder Zinnoxid (SnO), die ehemals von Atofina und gegenwärtig von Arkema vermarktet werden.

Vorzugsweise wird die Veresterung bei Temperaturen zwischen 100 - 300°C, insbesondere 200 - 250°C durchführt.

In einer weiteren Ausführungsform wird als Katalysator mindestens ein Enzym eingesetzt. Als Enzym eignen sich alle dem Fachmann bekannten Enzyme oder Enzymmischungen, welche in der Lage sind die Veresterung von Alkohol und Säure zu katalysieren, exemplarisch seinen genannt Lipasen, Acyltransferasen und Esterasen. Die enzymatisch katalysierte Veresterung wird üblicherweise bei Temperaturen von 20 bis 100 °C, vorzugsweise 40 bis 80 °C durchgeführt.

Gegenstand der Erfindung ist ein Verfahren zur Herstellung von Estern von 3-Methyl-2-propylhexanol mit Benzoesäure und/oder Benzoesäurederivaten, wobei man eine Mischung enthaltend 3-Methyl-2-Propylhexanol und den Methylester der entsprechenden Säure unter Zusatz eines Umesterungskatalysators umsetzt. Das erfindungsgemäße Verfahren umfasst ebenfalls die Herstellung von gemischten Estern, bei dem man 3-Methyl-2-Propylhexanol und einen weiteren Alkohol zusammen mit dem Methyl- oder Dimethylester der entsprechenden Dicarbonsäure unter Zusatz eines Umesterungskatalysators umsetzt.

Gegenstand der Erfindung ist ein Verfahren zur Herstellung von Estern von 4-Methyl-2-propylhexanol mit Benzoesäure und/oder Benzoesäurederivaten, wobei man eine Mischung enthaltend 4-Methyl-2-Propylhexanol und den Methylester der entsprechenden Säure unter Zusatz eines Umesterungskatalysators umsetzt. Das erfindungsgemäße Verfahren umfasst ebenfalls die Herstellung von gemischten Estern, bei dem man 4-Methyl-2-Propylhexanol und einen weiteren Alkohol zusammen mit dem Methyl- oder Dimethylester der entsprechenden Dicarbonsäure unter Zusatz eines Umesterungskatalysators umsetzt.

Gegenstand der Erfindung ist ein Verfahren zur Herstellung von Estern von 5-Methyl-2-propylhexanol mit Benzoesäure und/oder Benzoesäurederivaten, wobei man eine Mischung enthaltend 5-Methyl-2-Propylhexanol und den Methylester der entsprechenden Säure unter Zusatz eines Umesterungskatalysators umsetzt. Das erfindungsgemäße Verfahren umfasst ebenfalls die Herstellung von gemischten Estern, bei dem man 5-Methyl-2-Propylhexanol und einen weiteren Alkohol zusammen mit dem Methyl- oder Dimethylester der entsprechenden Dicarbonsäure unter Zusatz eines Umesterungskatalysators umsetzt.

Das erfindungsgemäße Verfahren umfasst ebenfalls die Herstellung von Estergemischen, bei dem man den Alkohol (5-Methyl-2-propylhexanol, 4-Methyl-2-propylhexanol, 3-Methyl-2-propylhexanol) zusammen mit den entsprechenden Mischungen der Methylester der Säuren unter Zusatz eines Umesterungskatalysators umsetzt.

In einer bevorzugten Ausführungsform wird die Mischung enthaltend Alkohol (5-Methyl-2-propylhexanol, 4-Methyl-2-propylhexanol, 3-Methyl-2-propylhexanol) und den Methylester der entsprechende Säure unter Zusatz des Veresterungskatalysators erhitzt, das entstehende Wasser kontinuierlich abgeführt und das Rohprodukt danach destilliert. In einer bevorzugten Ausführungsform wird das Verfahren ohne Zusatz von Lösungsmitteln durchgeführt, vorzugsweise mit Edukten, die möglichst wasserfrei sind.

Vorzugsweise wird die Veresterung bei Temperaturen zwischen 100- 300 °C, insbesondere 200 - 250°C durchführt. Als Umesterungskatalysator können alle dem Fachmann bekannten Umesterungskatalysatoren eingesetzt, werden, vorzugsweise wird als Umesterungskatalysator Natriummethylat oder Tetraalkyltitanat eingesetzt.
In einer weiteren Ausführungsform wird als Katalysator mindestens ein Enzym eingesetzt. Als Enzym eignen sich alle dem Fachmann bekannten Enzyme oder Enzymmischungen, welche in der Lage sind die Umesterung von Alkohol und Säuremethylester zu katalysieren, exemplarisch seinen genannt Lipasen, Acyltransferasen und Esterasen. Die enzymatisch katalysierte Veresterung wird üblicherweise bei Temperaturen von 20 bis 100 °C, vorzugsweise 40 bis 80 °C durchgeführt.

### Kosmetische/pharmazeutische Zubereitungen

Die 2-Propylheptylester der Benzoesäure und/oder Benzoesäurederivaten erlauben die Herstellung stabiler kosmetischer und pharmazeutischer Emulsionen mit besonders leichtem Hautgefühl.

Ein weiterer Gegenstand der vorliegenden Erfindung sind daher kosmetische und/oder pharmazeutische Zubereitungen enthaltend
(a) wenigstens einen Ester des 2-Propylheptanols mit Benzoesäure und/oder Benzoesäurederivaten
(b) wenigstens einen Emulgator und/oder Tensid und/oder Wachskomponente und/oder Polymer und/oder einen weiteren Ölkörper.

Vorzugsweise enthalten die erfindungsgemäßen Zubereitungen 0,1 bis 80, insbesondere 0,5 bis 70, vorzugsweise 0,75 bis 60 Gew.-%, insbesondere 1 bis 50 Gew.-%, bevorzugt 1 - 40 Gew.-% wenigstens eines Esters des 2-Propylheptanols mit Benzoesäure und/oder Benzoesäurederivaten. Ein weiterer Gegenstand der Erfindung sind Zubereitungen kosmetische und/oder pharmazeutische Zubereitungen enthaltend
a) 0,1 - 80 Gew.-%, insbesondere 0,1 bis 70, bevorzugt 0,1 bis 60, insbesondere 0,1 bis 50 Gew.-%, bevorzugt 0,1 - 40 Gew.-% wenigstens eines Ester des 2-Propylheptanols mit Benzoesäure und/oder Benzoesäurederivaten b) 0,1 - 20 Gew.-% Emulgator (b-1) und/oder Tensid (b-2) und/oder Wachskomponente (b-3) und/oder Polymer (b-4), 0,1 - 40 Gew.-% weiterer Ölkörper (b-5) und
c) 0 - 98 Gew.-% Wasser.

Die erfindungsgemäßen Zubereitungen enthalten mindestens 0,1, insbesondere mindestens 0,5, insbesondere mindestens 0,75, bevorzugt mindestens 1, bevorzugt mindestens 5 Gew.-% eines oder mehrerer Ester (a).

Alle Gew.-% Angaben beziehen sich auf Gew.-% bezogen auf die kosmetische und/oder pharmazeutische Zubereitung.

In einer Ausführungsform der Erfindung enthalten die Zubereitungen mindestens einen Ester ausgewählt aus der Gruppe bestehend aus 2-Propylheptyl-benzoesäureester, Di-[2-Propylheptyl-1,4-benzoldicarbonsäurediester, 2-Propylheptyl-1,4-benzolddicarbonsäureester, 2-Propylheptyl-2-Hydroxybenzoesäureester, 2-Propylheptyl-2-methylbenzoesäureester, 2-Propylheptyl-3-methylbenzoesäureester, 2-Propylheptyl-4-methylbenzoesäureester, 2-Propylheptyl-2-Acetoxylbenzoesäureester (=2-Propylheptyl-acetylsalicylsäureester).

Eine weitere bevorzugte Ausführungsform der kosmetischen und/oder pharmazeutischen Zubereitungen enthält (a) 0,1- 80, insbesondere 0,1 bis 70, vorzugsweise 0,1 bis 60, bevorzugt 0,1 bis 50 Gew.-% wenigstens eines Esters des 2-Propylheptanols mit Benzoesäure und/oder Benzoesäurederivaten, (b) 0,1 - 20 Gew.-% Emulgatoren (b-1) und/oder Tenside (b-2) und/oder Wachskomponenten (b-3) und/oder Polymere (b-4), und 0,1 - 40 Gew.-% weiterer Ölkörper (b-5) und (c) 0 - 98 Gew.-% Wasser.

Ein weiterer Gegenstand der vorliegenden Erfindung sind kosmetische und/oder pharmazeutische Zubereitungen enthaltend
(a) wenigstens einen Ester des 3-Methyl-2Propyl-Hexanols mit Benzoesäure und/oder Benzoesäurederivaten
(b) wenigstens einen Emulgator und/oder Tensid und/oder Wachskomponente und/oder Polymer und/oder einen weiteren Ölkörper.

Ein weiterer Gegenstand der vorliegenden Erfindung sind kosmetische und/oder pharmazeutische Zubereitungen enthaltend
(a) wenigstens einen Ester des 4-Methyl-2Propyl-Hexanols mit Benzoesäure und/oder Benzoesäurederivaten
(b) wenigstens einen Emulgator und/oder Tensid und/oder Wachskomponente und/oder Polymer und/oder einen weiteren Ölkörper.

Ein weiterer Gegenstand der vorliegenden Erfindung sind kosmetische und/oder pharmazeutische Zubereitungen enthaltend
(a) wenigstens einen Ester des 5-Methyl-2Propyl-Hexanols mit Benzoesäure und/oder Benzoesäurederivaten
(b) wenigstens einen Emulgator und/oder Tensid und/oder Wachskomponente und/oder Polymer und/oder einen weiteren Ölkörper.

Die erfindungsgemäßen Zubereitungen, die erfindungsgemäßen Zusammensetzungen sowie die Ester gemäß der Erfindung eignen sich als Basis in allen kosmetischen Mittel zur Körperpflege und -reinigung wie z.B. Körperöl, Babyöl, Körpermilch, Cremes, Lotionen, sprühbare Emulsionen, Sonnenschutzmittel, Antitranspirantien, Flüssig- und Stückseifen etc. eingearbeitet werden. Sie lassen sich auch in tensidhaltigen Formulierungen wie z.B. Schaum- und Duschbädem, Haarshampoos und Pflegespülungen einsetzen. Sie lassen sich als Pflegekomponente auf Tissues, Papiere, Wipes, Vlies-Produkte, Schwämme, Puffs, Pflaster und Bandagen applizieren, die ihren Einsatz im Bereich der Hygiene und Pflege finden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte, Selbstbräunungs Wipes). Sie lassen sich u.a. auch in Zubereitungen zur Haarpflege, Haarreinigung oder Haarfärbung einsetzen.

Je nach Applikationszweck enthalten die kosmetischen Formulierungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

### Emulgator b-1)

In einer Ausführungsform der Erfindung enthalten die erfindungsgemäßen Zubereitungen mindestens einen Emulgator. Die erfindungsgemäßen Zusammensetzungen enthalten den/die Emulgator(en) in einer Menge von 0 bis 40 Gew.-%, vorzugsweise 0,1 bis 20 Gew.-% vorzugsweise 0,1 bis 15 Gew.-% und insbesondere 0,1 bis 10 Gew.-% bezogen auf das Gesamtgewicht der Zusammensetzung.

In einer Ausführungsform der Erfindung enthält die erfindungsgemäße Zubereitung mehr als einen Emulgator. Der Fachmann setzt in Abhängigkeit der übrigen Komponenten übliche Emulgator Systeme (wie z.B. Emulgator und Co-Emulgator) ein.

### Nicht-ionische Emulgatoren

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂-C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester wie z. B. Glycerylstearatcitrat und Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls^{®} PGPH" (W/O-Emulgator) oder "Eumulgin^{®}VL 75" (Abmischung mit Coco Glucosides im Gewichtsverhältnis 1:1, OIW-Emulgator) oder Dehymuls^{®} SBL (W/O-Emulgator) von der Cognis Deutschland GmbH vertrieben werden. In diesem Zusammenhang sei insbesondere auf das Europäische Patent EP 0 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer, "Encyclopedia of Chemical Technology", 3. Aufl., 1979, Band 8, Seite 913, aufgelistet. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestem, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycolether), sowie Eumulgin^{®} SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade^{®} PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin^{®} VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Tenside b-2)

In einer Ausführungsform der Erfindung enthalten die erfindungsgemäßen Zubereitungen mindestens ein Tensid. Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Die erfindungsgemäßen Zusammensetzungen enthalten den/die Tenside in einer Menge von 0 bis 40 Gew.-%., vorzugsweise 0 bis 20 Gew.-%, vorzugsweise 0,1 bis 15 Gew.-% und insbesondere 0,1 bis 10 Gew.-% bezogen auf das Gesamtgewicht der Zusammensetzung.

Typische Beispiele für nichtionische Tenside sind Feftalkoholpolyglycolether, Alkylphenolpolyglycolether, Feftsäurepolyglycolester, Fettsäureamidpolyglycolether, Feftamin-polyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

**Anionische Tenside** sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Als **kationische Tenside** sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quatemierte Fettsäuretriethanolaminestersalze verstanden. Sie können den erfindungsgemäßen Zusammensetzungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quatemisierten Proteinhydrolysate dar.

### Wachskomponente b-3)

In einer Ausführungsform der Erfindung enthalten die erfindungsgemäßen Zubereitungen mindestens eine Wachskomponente. Die erfindungsgemäßen Zusammensetzungen enthalten den/die Wachskomponente(n) in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zusammensetzung.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30 C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.
Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten Triglycerid-Gemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata AB und Novata B (Gemisch aus C12-C18-Mono-, Di- und Triglyceriden) sowie Cutina MD oder Cutina GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Fettalkohole, die beispielsweise von der Cognis Deutschland GmbH unter der Bezeichnung Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zusammensetzungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar.

### Polymere b-4)

In einer Ausführungsform der Erfindung enthalten die erfindungsgemäßen Zubereitungen mindestens ein Polymer. Die erfindungsgemäßen Zusammensetzungen enthalten das/die Polymere(n) in einer Menge von 0 bis 20 Gew.-%, vorzugsweise 0,1 bis 15 Gew.-% und insbesondere 0,1 bis 10 Gew.-% bezogen auf das Gesamtgewicht der Zusammensetzung.

Geeignete **kationische Polymere** sind beispielsweise kationische Cellulosederivate, wie z. B. eine quatemierte Hydroxyethylcellulose, die unter der Bezeichnung Polymer JR 400^{®} von Amerchol erhältlich ist, kationische Stärke, Copolymere von Diallylammoniumsalzen und Acrylamiden, quatemierte VinylpyrrolidonNinylimidazol-Polymere, wie z.B. Luviquaf (BASF), Kondensationsprodukte von Polyglycolen und Aminen, quatemierte Kollagenpolypeptide, wie beispielsweise Lauryldimonium hydroxypropyl hydrolyzed collagen (Lamequat^{®}L/Grünau), quatemierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar^{®} CBS, Jaguar^{®} C-17, Jaguar^{®} C-16 der Firma Celanese, quatemierte Ammoniumsalz-Polymere, wie z. B. Mirapol^{®} A-15, Mirapol^{®} AD-1, Mirapol^{®} AZ-1 der Firma Miranol.

Als **anionische, zwitterionische, amphotere und nichtionische Polymere** kommen beispielsweise Vinylacetat/Crotonsäure-Copolymere, VinylpyrrolidonNinylacrylat-Copolymere, Vinylacetat/Butylmaleat/ Isobornylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ DimethylaminoethylmethacrylatlVinylcaprolactam-Terpolymere sowie gegebenenfalls derivatisierte Celluloseether und Silicone in Frage.

Als Polymere eigen sich ebenso Polysaccharide, insbesondere Xanthan-Gum, Guar-Guar, Agar-Agar, Alginate und Tylosen.

### Weitere Ölkörper b-5)

Körperpflegemittel, wie Cremes, Körperöle, Lotionen und Milchen, enthalten üblicherweise eine Reihe weiterer Ölkörper und Emollients, die dazu beitragen, die sensorischen Eigenschaften weiter zu optimieren. Die Ölkörper (erfindungsgemäße Ester plus weitere Ölkörper) sind üblicherweise in einer Gesamtmenge von 0,1 -80, insbesondere 0,5 bis 70, bevorzugt 1 bis 60, insbesondere 1 bis 50 Gew.-%, insbesondere 1 bis 40 Gew.-%, vorzugsweise 5 - 25 Gew.-% und insbesondere 5 - 15 Gew.-% enthalten. Die weiteren Ölkörper sind üblicherweise in einer Menge von 0,1 bis 40 Gew.-% enthalten

Als weitere Ölkörper kommen beispielsweise Guerbetalkohole auf Basis von Fettalkoholen mit 6 bis 18, vorzugsweise 8 bis 10 Kohlenstoffatomen, in Frage sowie weitere, zusätzliche Ester wie Myristyimyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestern mit Polyolen und Kohlenwasserstoffen oder deren Gemischen (Cetiol® DD).

### Weitere Inhaltsstoffe

Als **Verdickungsmittel** eignen sich beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z. B. 4-tert.-Butyl-4'-methoxydibenzoylmethan (Parsol^{®} 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wassedöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen auch unlösliche Lichtschutzpigmente, nämlich feindisperse Metalloxide in Frage. Beispiele für geeignete Metalloxide sind insbesondere Zinkoxid und Titandioxid. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als **Selbstbräuner** eignet sich Dihydroxyaceton. Als Tyrosinhinbitoren, die die Bildung von Melanin verhindern und Anwendung in Depigmentierungsmifteln finden, kommen beispielsweise Arbutin, Ferulasäure, Kojisäure, Cumarinsäure und Ascorbinsäure (Vitamin C) in Frage.

Als **Konservierungsmittel** eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Perlglanzwachse,** insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Stabilisatoren** können Metallsalze von Fettsäuren, wie z. B. Magnesium-, Aluminium- und/oder Zinkstearat bzw. -ricinoleat eingesetzt werden.

Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Die erfindungsgemäßen Zubereitungen, die erfindungsgemäßen Zusammensetzungen sowie die Ester gemäß der Erfindung eignen sich insbesondere in kosmetischen und/oder pharmazeutischen Zubereitungen zur Benetzung oder Imprägnierung oder Beschichtung von Gebrauchs- und Hygienetüchem, die zur Körperreinigung und/oder zur Körperpflege eingesetzt werden.

Als Gebrauchs- und Hygienetücher seien exemplarisch genannt: Tissues, Papiere, Wipes, Vlies-Produkte, Schwämme, Puffs, Pflaster und Bandagen, die ihren Einsatz im Bereich der Hygiene und Pflege finden. Dies können sein Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte sowie Selbstbräunungs-Wipes.

## Patentansprüche

1. Verwendung von Estern des 2-Propylheptanols mit Benzoesäure und/oder Benzoesäurederivaten in kosmetischen und/oder pharmazeutischen Zubereitungen.

2. Verwendung gemäß Anspruch 1, **dadurch gekennzeichnet dass** als Benzoesäurederivate Verbindungen eingesetzt werden ausgewählt aus der Gruppe, die gebildet wird von methylsubstituierter Benzoesäure, hydroxysubstituierter Benzoesäure, carboxysubstituierter Benzoesäure, durch Acylierung von hydroxysubstituierten Benzoesäuren erhältlichen Derivaten.

3. Ester des 2-Propylheptanols mit Benzoesäure und/oder Benzoesäurederivaten.

4. Ester nach Anspruch 3, ausgewählt aus der Gruppe bestehend aus 2-Propylheptyl-benzoesäureester, Di-[2-Propylheptyl-1,4-benzoldicarbonsäurediester, 2-Propylheptyl-1,4-benzoldicarbonsäureester, 2-Propylheptyl-2-Hydroxybenzoesäureester, 2-Propylheptyl-2-methylbenzoesäureester, 2-Propylheptyl-3-methylbenzoesäureester, 2-Propylheptyl-4-methylbenzoesäureester, 2-Propylheptyl-2-Acetoxylbenzoesäureester (=2-Propylheptyl-acetylsalicylsäureester).

5. Zusammensetzungen, enthaltend einen Ester von 2-Propylheptanol mit Benzoesäure und/oder Benzoesäurederivaten und mindestens einen weiteren Ester derselben Säure mit einem Alkohol ausgewählt aus der Gruppe bestehend aus 3-Methyl-2-Propylhexanol, 4-Methyl-2-Propylhexanol oder 5-Methyl-2-Propylhexanol.

6. Verfahren zur Herstellung der Ester gemäß einem der vorgenannten Ansprüche, wobei man eine Mischung enthaltend 2-Propylheptanol und die entsprechende Säure umsetzt.

7. Verfahren zur Herstellung der Ester gemäß einem der vorgenannten Ansprüche, wobei man eine Mischung enthaltend 2-Propylheptanol und den Methylester der entsprechenden Säure unter Zusatz eines Umesterungskatalysators umsetzt.

8. Kosmetische und/oder pharmazeutische Zusammensetzungen enthaltend
(a) wenigstens einen Ester des 2-Propylheptanols mit Benzoesäure und/oder Benzoesäurederivaten
(b) wenigstens einen Emulgator (b-1) und/oder Tensid (b-2) und/oder Wachskomponente (b-3) und/oder Polymer (b-4) und/oder einen weiteren Ölkörper (b-5).

9. Zusammensetzung gemäß Anspruch 8, enthaltend 0,1 - 80 Gew.-%, insbesondere 0,1 bis 70, bevorzugt 0,1 bis 60, insbesondere 0,1 bis 50 Gew.-%, bevorzugt 0,1 - 40 Gew.-% wenigstens eines Ester des 2-Propylheptanols mit Benzoesäure und/oder Benzoesäurederivaten.

10. Zusammensetzung gemäß Anspruch 8 und/oder 9 enthaltend
a) 0,1 - 80 Gew.-%, insbesondere 0,1 bis 70, bevorzugt 0,1 bis 60, insbesondere 0,1 bis 50 Gew.-%, bevorzugt 0,1 - 40 Gew.-% wenigstens eines Ester des 2-Propylheptanols mit Benzoesäure und/oder Benzoesäurederivaten
b) 0,1 - 20 Gew.-% Emulgator (b-1) und/oder Tensid (b-2) und/oder Wachskomponente (b-3) und/oder Polymer (b-4), 0,1-40 Gew.-% weiterer Ölkörper (b-5) und
c) 0- 98 Gew.-% Wasser.
